# EUROPEAN PATENT APPLICATION

(11) **EP 1 358 871 A1**
(43) Date of publication of application: **05.11.2003**
(21) Application number: 02729563.3
(22) Date of filing: 11.01.2002
(51) Int. Cl.: A61K 7/50

(54) **BATH ADDITIVE COMPOSITION**

(30) Priority: 16.01.2001 JP 2001007589
(71) Applicant: TSUMURA & CO., Chuo-ku, Tokyo 103-0027 (JP)
(72) Inventor: NAKANISHI, Nobuyuki, Tsumura & Co., Inashiki-gun, Ibaraki 300-1192 (JP); WATANABE, Satoshi, Tsumura & Co., Inashiki-gun, Ibaraki 300-1192 (JP)
(74) Representative: Woods, Geoffrey Corlett
(86) International application number: JP0200156
(87) International publication number: WO02055054

(57) **Abstract**

The present invention relates to a bath additive composition comprising (A) at least one α-hydroxy acid, and (B) at least one member selected from among ascorbic acid, a derivative thereof and mulberry bark (Mori Cortex) extract.

## Description

### TECHNICAL FIELD

The present invention relates to a bath additive composition which can gently eliminate aged horny substances over the entire body, and which can conveniently improve skin color over the entire body.

### BACKGROUND ART

Conventionally, creams and lotions exist in which α-hydroxy acid and a whitening component are formulated, and which are expected to effect an improvement of skin condition and a whitening action. However, these creams and lotions, in seeking this effect, had α-hydroxy acid formulated at high concentration which causes problems such as skin trouble and low transdermal absorptivity of the whitening component, and thus were unsatisfactory. Further, creams or lotions, given the particular characteristics of the formulation thereof, can only be used locally and thus improvement of skin color over the entire body cannot be obtained therefrom.

### DISCLOSURE OF THE INVENTION

An object of the present invention is to provide a composition that can gently eliminate aged horny substances over the entire body, increase transdermal absorptivity of a whitening component, and thereby enable simple improvement of skin color over the entire body.

The present inventors, as a result of deliberate research conducted in order to achieve the above object, found that by exploiting a synergetic effect of the horny substance stripping action of α-hydroxy acid, and skin swelling and hydrostatic action when bathing, it is possible to gently eliminate aged horny substances from over the entire body, increase transdermal absorptivity of a whitening component, thereby allowing simple improvement of skin color over the entire body, and thus completed the present invention.

That is, the present invention includes the following content:
(1) A bath additive composition comprising (A) at least one α-hydroxy acid, and (B) at least one member selected from among ascorbic acid, a derivative thereof and mulberry bark (Mori Cortex) extract.
(2) A bath additive composition according to item (1) above, wherein the at least one α-hydroxy acid is selected from among malic acid, lactic acid, glycolic acid and citric acid.

The present invention is explained in detail below.

In the bath additive composition of the present invention, (A) the α-hydroxy acid component acts as a horny substance eliminating agent, and (B) the ascorbic acid, derivative thereof or mulberry bark (Mori Cortex) extract component acts as a whitening component.

The α-hydroxy acid used in the present invention is a compound which has a structure wherein there is at least 1 carboxyl group and hydroxyl group respectively within a molecule thereof, and at least 1 carboxyl group and at least 1 hydroxyl group are bound by an identical carbon atom.

Examples of the α-hydroxy acid include compounds having 1 carboxyl group and 1 hydroxyl group, such as glycolic acid, lactic acid and the like; compounds having two carboxyl groups and 1 hydroxyl group such as malic acid; and compounds having 3 carboxyl groups and 1 hydroxyl group such as citric acid.

Examples of a derivative of ascorbic acid include, sodium ascorbate, ascorbyl esters of fatty acids, magnesium ascorbyl phosphate, disodium ascorbyl sulfate and the like.

Combined use of ascorbic acid and mulberry bark (Mori Cortex) extract as the whitening component (B), is preferable

The amount of α-hydroxy acid to be formulated in the bath additive composition of the present invention is normally, 0.01 to 50% by weight, and preferably 0.05 to 30% by weight.

The amount of the whitening component (B) (at least one member selected from among ascorbic acid, a derivative thereof, and mulberry bark (Mori Cortex) extract) to be formulated in the bath additive composition of the present invention, is normally 0.001 to 20% by weight, and preferably 0.01 to 10% by weight.

The formulation ratio of α-hydroxy acid (A) to the whitening component (B) [(A):(B)] is normally 1:2000 to 50000:1, and preferably 1:200 to 3000:1.

In the bath additive composition of the present invention, a whitening agent other than the aforementioned whitening agent (B), for example, placenta extract, licorice extract, or the like, can be further formulated therein.

Further, components other than the above components, such as an inorganic pigment, moisturizer, surfactant, preservative, fragrance, inorganic salt, oil component, vitamin, proteolytic enzymes, and other components can be added to the bath additive composition of the present invention, as required.

Examples of an inorganic pigment include titanium oxide, calcium carbonate, magnesium carbonate, zinc oxide (zinc white), talc, calcium silicate, anhydrous silicic acid, kaolin, bentonite, mica titanium, and the like.

Examples of a moisturizer include saccharides such as sorbitol, xylitol, maltitol, dextrin, glucose, maltose, lactose, sucrose, xylose, fructose, mannitol, lactitol and the like; multivalent alcohols such as ethylene glycol, propylene glycol, 3-methyl-1,3-butane diol, glycerol, 1,3-butylene glycol and the like; amino acids such as sodium pyrrolidone carboxylate; and urea and the like.

Examples of a surfactant include non-ion surfactants such as glycerol fatty acid ester, propylene glycol fatty acid ester, sorbitan fatty acid ester, polyoxyethylene sorbitan fatty acid ester, polyoxyethylene sorbitol tetraoleate, polyoxyethylene alkyl ether, polyoxyethylene polyoxypropylene alkyl ether, polyethylene glycol fatty acid ester, polyoxyethylene hardened castor oil, polyglycerol fatty acid ester, and the like; anion surfactants such as sodium α-olefin sulfonate, sodium lauryl sulfate, sodium cetyl sulfate, polyoxyethylene sodium lauryl sulfate, disodium lauryl sulfosuccinate and the like; amphoteric surfactants such as carboxybetaine-type, amino carboxylic acid, sulfobetaine-type and the like; and cation surfactants. However from the viewpoint of safety, a non-ion surfactant is preferable.

Examples of a preservative include paraoxybenzoic acid ester (for example, methyl paraben), benzoic acid, benzoate, and phenoxyethanol.

Examples of a fragrance include natural fragrances such as lavender oil, jasmine oil, lemon oil, and the like; and synthetic fragrances such as geraniol, citronellol, phenethyl alcohol and the like.

Examples of inorganic salts include sodium sulfate, magnesium sulfate, sodium chloride, potassium chloride, sodium carbonate, sodium bicarbonate, sodium sesquicarbonate, and sodium thiosulfate.

Examples of an oil component include fats and oils such as natural fats and oils such as soy bean oil, rice bran oil, jojoba oil, avocado oil, almond oil, olive oil, cocoa oil, sesame seed oil, apricot kernel oil, castor oil, palm oil, mink oil, beef tallow, lard, and the like, hardened oils obtained by hydrogenation of these natural fats and oils, synthetic glycerides such as glyceride of myristic acid, glyceride of 2-ethylhexanoic acid, and the like, diglycerides and the like; waxes such as carnauba wax, whale wax, beeswax, lanoline and the like; hydrocarbons such as liquid paraffin, vaseline, paraffin, microcrystalline wax, ceresin, squalane, pristane and the like; higher fatty acids such as lauric acid, myristic acid, palmitic acid, stearic acid, behenic acid, oleic acid, linolic acid, linolenic acid, lanolinic acid, isostearic acid and the like; higher alcohols such as lauryl alcohol, cetyl alcohol, stearyl alcohol, oleyl alcohol, cholesterol, 2-hexyldecanol and the like; esters such as cetyl octanoate, myristyl lactate, cetyl lactate, isopropyl myristate, isopropyl palmitate, isopropyl adipate, butyl stearate, decyl oleate, cholesterol isostearate and the like; essential oils; and silicone oils.

Examples of vitamins include vitamin A, vitamin B, vitamin D, vitamin E, vitamin F, vitamin K, vitamin P, vitamin U, carnitine, ferulic acid, γ-oryzanol, lipoic acid, orotic acid and a derivative thereof. Examples of a proteolytic enzyme include pepsin, trypsin, chymotrypsin, cathepsin, papain, bromelain, ficin, proteases derived from bacteria, yeast or mold, and the like.

Examples of other components include sulfur, mineral sand, flowers of sulfur, neutral clay, egg yolk powder, roasted rice bran, mica powder, skim milk powder, seaweed extract, pigment, disinfectant and the like.

The bath additive composition of the present invention may be in the form of a powder, granule, tablet or liquid.

This specification includes part or all of the contents as disclosed in the specification of Japanese Patent Application 2001-7589, which is the base of the priority claim of the present application.

### BEST MODE FOR CARRYING OUT THE INVENTION

Below, the present invention is explained in detailed by means of Examples. However, these Examples do not limit the present invention in any way.

### (Examples 1 to 2 and Comparative Examples 1 to 4)

Various formulations of the compositions shown in Table 1 were manufactured. The bath additives of Example 1 and Comparative Examples 1 and 2 are bath additives in powder form, and the bath additive of Example 2 is a bath additive in liquid form.

Skin color improvement effect and skin irritation were evaluated in accordance with the following methods in respect of all of the formulations.

### <Skin color improvement effect>

Skin color improvement effect (ΔL value=L₃₀-L₀) was determined using a commercially available hand-held colorimeter for each of the formulations of Examples 1 to 2 and comparative Examples 1 to 4, by measuring L₃₀ value (lightness) at 30 minutes after bathing or application and in each case comparing with an L₀ value prior to bathing or application. In respect of the bath additives, a whole body bath was conducted for 10 minutes at 40±1ºC, with each formulation at 50g/200L. Experimental subjects were 6 male and 9 female, a total of 15, healthy individuals for each formulation. Results are shown in Table 1.

### <Skin irritation>

After the experiment on skin color improvement effect, all subjects were questioned in respect of irritation experienced, and average values were determined using the following scoring. Results are shown in Table 1.
(Score) 3: substantial irritation experienced, 2: irritation experienced, 1: slight irritation experienced, 0: no irritation experienced.

**Table 1**

| Component | | Example 1 | Comparative Example I | Comparative Example 2 | Comparative Example 3 | Example 2 | Comparative Example 4 |
|---|---|---|---|---|---|---|---|
| | | Bath additive | Bath additive | Bath additive | Lotion | Bath additive | Cream |
| α-hydroxy acid | lactic acid | | | | | 2 | 2 |
| | malic acid | 5 | 5 | | 5 | | |
| whitening component | ascorbic acid | 3 | | | 3 | | |
| | magnesium ascorbyl phosphate | | | | | 1.5 | 1.5 |
| | mulberry bark extract | 1 | | 1 | 1 | | |
| sodium bicarbonate | | 50 | 50 | 50 | | | |
| sodium sulfate | | 40 | 44 | 45 | | | |
| ethanol | | | | | 8 | 20 | |
| 1,3-butylene glycol | | | | | | 10 | 5 |
| concentrated glycerol | | | | | 20 | 5 | |
| POE hardened castor oil | | | | | 0.2 | 0.2 | |
| squalane | | | | | | | 20 |
| stearic acid | | | | | | | 8 |
| polyethylene glycol | | | | | 1.5 | | 10 |
| glyceryl monostearate | | | | | | | 3 |
| POE cetyl ether | | | | | | | 5 |
| purified water | | | | | 60 | 60 | 45 |
| dibutylhydroxytoluene | | | | | appropriate amount | appropriate amount | appropriate amount |
| methyl paraben | | | | | appropriate amount | appropriate amount | appropriate amount |
| fragrance | | appropriate amount | appropriate amount | appropriate amount | trace amount | appropriate amount | trace amount |
| pigment | | trace amount | trace amount | trace amount | | trace amount | |
| Total | | 100 | 100 | 100 | 100 | 100 | 100 |
| skin color improvement effect (ΔL value) | | 1.17 | 0.35 | 0.42 | 0.13 | 1.06 | 0.22 |
| Skin irritation (average score) | | 0.1 | 0.2 | 0 | 2.1 | 0.1 | 1.7 |

All publications, patents and patent applications cited herein are incorporated herein by reference in their entirety.

### INDUSTRIAL APPLICABILITY

The composition of the present invention can gently eliminate aged horny substances from over the entire body, and can increase transdermal absorptivity of a whitening component thereby enabling simple improvement of skin color over the entire body.

## Claims

1. A bath additive composition comprising (A) at least one α-hydroxy acid, and (B) at least one member selected from among ascorbic acid, a derivative thereof and mulberry bark (Mori Cortex) extract.

2. A bath additive composition according to claim 1 wherein the at least one α-hydroxy acid is selected from among malic acid, lactic acid, glycolic acid and citric acid.
